# EUROPEAN PATENT APPLICATION

(11) **EP 4 699 480 A1**
(43) Date of publication of application: **25.02.2026**
(21) Application number: 24195910.5
(22) Date of filing: 22.08.2024
(51) Int. Cl.: A24F 42/20, A24F 42/60, A61M 15/06

(54) **SUBSTANCE CHAMBER AND INHALATOR COMPRISING A SUBSTANCE CHAMBER**

(71) Applicant: ClkGroupDenmark, 3730 Nexø (DK)
(72) Inventor: KNUDSEN, Carsten Leonhard, 3730 Nexø (DK)
(74) Representative: COPA Copenhagen Patents

(57) **Abstract**

Substance chamber for an inhalator comprising the substance chamber and an evaporation chamber, wherein the substance chamber comprises a first cylindrical shell extending in a longitudinal axis from a first end to a second end, and having an inner diameter, an outer diameter, and a wall thickness being half of the difference between the inner diameter and the outer diameter. The substance chamber further comprising a first sealing body and a second sealing body movably arranged inside the first cylindrical shell, wherein the first cylindrical shell, the first sealing body and the second sealing body forms a substance compartment containing at least a primary substance, preferably a liquid substance.

## Description

The present invention relates to an improved inhalator, inhalator parts and a method of manufacturing an inhalator.

### BACKGROUND

Various types of inhalators are known and may be used to substitute the use of cigarettes. However, such inhalators comprise various drawbacks. For example, some inhalators require use of heat, e.g. provided by electrical heating, to vaporise the substances inside the inhalator. Also manufacturing of inhalators are often complex and time-consuming. Furthermore, the weight of a typical inhalator is much higher than a conventional cigarette. There is therefore also a desire to reduce the weight of inhalators to closer resemble the feel of a conventional cigarette.

### SUMMARY

It is an object of the present disclosure to facilitate an improved inhalator which is easier and faster to manufacture. Particularly, the present disclosure provides an inhalator comprising a plurality of inhalator parts.

Accordingly, a substance chamber for an inhalator, such as an inhalator comprising the substance chamber and an evaporation chamber, is disclosed. The substance chamber comprises a first cylindrical shell extending in a longitudinal axis from a first end to a second end. The substance chamber has an inner diameter, an outer diameter, and a wall thickness. The wall thickness is half of the difference between the outer diameter and the inner diameter.

The substance chamber comprises a first sealing body and a second sealing body. The first sealing body and the second sealing body are movably arranged inside the first cylindrical shell. The first cylindrical shell, the first sealing body and the second sealing body form a substance compartment containing a substance, such as at least a primary substance. The substance, such as the primary substance, is preferably a liquid substance.

Often inhalators are manufactured as a single tube where each component or part of the inhalator is inserted into the tubing in a specific order. This method of manufacturing an inhalator is complex and time-consuming.

Furthermore, the tube which the component is inserted into constitutes a weight which is higher than the conventional cigarettes. There is therefore a desire to reduce the weight of inhalators to closer resemble the feel of a conventional cigarette.

Thus, an inhalator comprising a plurality of inhalator parts is also disclosed. The inhalator extends from a first end to a second end along a longitudinal axis. The plurality of inhalator parts comprises a substance chamber according to the present disclosure, as mentioned above. The longitudinal axis of the first cylindrical shell and the longitudinal axis of the inhalator are coinciding and/or the longitudinal axis of the first cylindrical shell is the longitudinal axis of the inhalator. The plurality of inhalator parts comprises an evaporation chamber. The evaporation chamber comprises a second cylindrical shell extending along the longitudinal axis from a first end to a second end. An inner diameter of the second cylindrical shell is larger than the inner diameter of the first cylindrical shell of the substance chamber. The substance chamber and the evaporation chamber are arranged coaxially along the longitudinal axis. The first end of the first cylindrical shell is arranged towards the second end of the second cylindrical shell.

Also disclosed is a method for manufacturing an inhalator comprising a plurality of inhalator parts and extending from a first end to a second end along a longitudinal axis. The method comprises providing the plurality of inhalator parts comprising providing a first substance chamber according to the substance chamber, as mentioned above. The longitudinal axis of the first cylindrical shell and the longitudinal axis of the inhalator are coinciding and/or the longitudinal axis of the first cylindrical shell is the longitudinal axis of the inhalator.

Providing the plurality of inhalator parts may comprise providing a first evaporation chamber, such as the evaporation chamber as mentioned above, comprising a second cylindrical shell extending along the longitudinal axis from a first end to a second end. An inner diameter of the second cylindrical shell is larger than the inner diameter of the first cylindrical shell of the substance chamber.

The method comprises arranging the first substance chamber and the first evaporation chamber coaxially along the longitudinal axis. The method comprises arranging the first substance chamber and the first evaporation chamber such that the first end of the first cylindrical shell is arranged towards the second end of the second cylindrical shell.

The method comprises providing a wrapping sheet, e.g. comprising adhesive on a first surface. The method comprises wrapping the wrapping sheet circumferentially around the longitudinal axis and such that the first surface is facing and encircling the first substance chamber and the first evaporation chamber. The adhesive may be applied to the wrapping sheet in an area which may cover the first end of the first cylindrical shell and the second end of the second cylindrical shell.

The evaporation chamber may comprise one or more pellets. The pellets may increase the surface area within the evaporation chamber and thereby improve evaporation of the substance, such as the primary substance, e.g. when advanced into the evaporation chamber. The pellets may be or may comprise grains, such as rice or wheat grains. The pellets may additionally or alternatively comprise or be seeds such as plant seeds, such as seeds from lavender, spices, strawberry, raspberry, liquorice root, alfalfa, clover, thyme, basil and/or other plants or a combination hereof. By providing pellets as seeds the inhalator may be used to provide plants in the nature after use. Particularly, the plant seeds may be seeds tolerant to the substance(s) in the substance chamber, such that the plant seeds after being in contact with the substance(s) from the substance chamber maintains its ability to grow and evolve into a plant.

The inhalator may further comprise a first filter extending from a first end to a second end along the longitudinal axis. The first filter and the first cylindrical shell may be arranged coaxially along the longitudinal axis and such that the first end of the first filter is arranged towards the second end of the first cylindrical shell.

A rod may be provided for activating the inhalator. Particularly, the rod may be configured to push, e.g., by an external force, the first sealing body and/or the second sealing body. The rod may be configured to push the first sealing body and/or the second sealing body from the substance chamber towards and/or into the evaporation chamber. The rod may form part of the inhalator. Alternatively, the rod may be provided separate to the inhalator.

The inhalator may comprise the rod extending from a first end to a second end along the longitudinal axis. The rod and the first filter may be arranged coaxially along the longitudinal axis. The first end of the rod may be arranged towards the second end of the first filter. The rod, e.g., the first end of the rod, may be configured to be received in the first filter. The rod may protrude through the first filter. The rod may be received in the first filter without protruding through the first filter.

The rod may comprise a rod body and a rod head. The rod head may comprise openings to allow air to pass through the rod head and through the inhalator. The length of the rod along the longitudinal axis may be larger than the length of the first cylindrical shell along the longitudinal axis. The length of the rod may be equal to or larger than the sum of the length of the first filter and the first cylindrical shell along the longitudinal axis. The length of the first filter may correspond to the extent from the first end of the filter to the second end of the filter along the longitudinal axis. The length of the substance chamber may correspond to the length of the first cylindrical shell. The length of the substance chamber may correspond to the extent from the first end of the first cylindrical shell to the second end of the first cylindrical shell along the longitudinal axis.

The method may comprise providing a plurality of inhalator parts where providing the plurality of inhalator parts may comprise providing a rod, such as the rod mentioned above, extending from a first end to a second end along the longitudinal axis. Providing the plurality of inhalator parts may comprise providing a first filter extending from a first end to a second end along the longitudinal axis and comprising a through hole. The rod may be received in the through hole of the first filter. The method may comprise arranging the first filter and the first substance chamber coaxially along the longitudinal axis, and such that the second end of the first cylindrical shell is arranged towards the first end of the first filter. The first filter may prevent leakage of the substance from the inhalator. The first filter may be provided as a holder for the rod. In the assembled inhalator the rod may be engaged in the first filter, or the rod may be provided as a separate part of the inhalator.

Further advantages of the present disclosure includes that an inhalator may be provided with reduced weight, e.g. more closely resembling the feel of a conventional cigarette weighing about 1 gram. Also, the present invention facilitates ease of production, enabling high-speed and high-volume manufacturing of an inhalator.

The inhalator may comprise a second filter extending from a first end to a second end along the longitudinal axis. The second filter and the second cylindrical shell may be arranged coaxially along the longitudinal axis. The second end of the second filter may be arranged towards the first end of the second cylindrical shell.

The second filter may comprise a first portion at the first end of the second filter and a second portion at the second end of the second filter. The first portion may comprise a through hole extending along the longitudinal axis. The second portion may be devoid of a though hole. The second portion may have a substantially continuous and/or uniform cross section. The first portion and/or second portion may be of a porous material, e.g., comprising a multi-cellular cross section, e.g., an open-celled cross-section. The second portion may allow air to pass through. The first portion may be of a porous material and may comprise a through hole.

The first portion of the second filter may increase the amount of evaporated substance for intake for the user by increasing the exposure area. The second portion may prevent the substance in liquid form and/or the pellets from escaping the evaporation chamber while allowing substance in evaporated form to pass. The second portion may also increase the amount of evaporated substance by increasing the exposure area for the substance.

The inhalator may comprise a wrapping sheet. The wrapping sheet may comprise a first surface facing the plurality of inhalator parts. The wrapping sheet may comprise an adhesive on the first surface. The wrapping sheet may be wrapped circumferentially around the longitudinal axis and encircling the substance chamber, the evaporation chamber, the first filter and/or the second filter. The wrapping sheet may be of a non-toxic material, such as paper. The adhesive may be a non-toxic adhesive, such as honey. The adhesive may adhere the wrapping sheet to the plurality of inhalator parts, such that the plurality of inhalator parts maintains their respective positions. Particularly, the adhesive may provide for maintaining the substance chamber and the evaporation chamber in their relative positions. For example, the adhesive may adhere the substance chamber and the evaporation chamber together. The adhesive may be provided on the first surface of the wrapping sheet at a position corresponding to the intersection between the substance chamber and the evaporation chamber.

An advantage of the present disclosure is that a more environmentally friendly inhalator may be provided. For example, biodegradable and/or non-toxic materials may be used to a larger extent, and possibly the present invention facilitates that an entire inhalator may be manufactured, which after being used can be composted as other food scraps. By providing the inhalator or the parts of the inhalator in biodegradable material, the environmental impact and contamination of the production and consumption of inhalators is reduced.

The plurality of inhalator parts, such as the rod, the first and/or the second filter and/or the wrapping sheet may be provided with seeds, such as plant seeds, such as seeds from lavender, spices, strawberry, raspberry, liquorice root, alfalfa, clover, thyme, basil and/or other plants or a combination hereof. This allows for plants or trees to grow after use and disposal of the inhalator. The seeds may be relatively small such that birds, microbes or snails may be able to eat them and such that they may be provided at one or more of the plurality of inhalator parts. The seeds may have antibacterial properties. The seeds may be of high nutrition.

One or more, or all of, the plurality of inhalator parts may be made of recycled material. For example, the wrapping sheet may be made of a biodegradable material, such as paper made of biodegradable material, such as hemp, rice, cellulose, algae or a combination hereof. The wrapping sheet may comprise two layers of wrapping sheets with seeds arranged between the two sheets.

The wrapping sheet may be provided with a first material to reduce flammability and enhance durability. The wrapping sheet may be saturated or coated with the first material. The first material may be lime, kaolin, clay, cellulose fibres, ammonium phosphates or other minerals, or a combination hereof.

The wrapping sheet may be coated with a second material. The second material may be of bioplastics, wax or other biodegradable polymers. The wrapping sheet may be coated with the second material on the first surface.

The adhesive used for the inhalator, e.g. the adhesive of the wrapping sheet, may be of a biodegradable material, such as biobased adhesive, such as plant-based starch or plant-based polymers, such as potato or corn, or a combination hereof. The adhesive may be provided as a mixture of adhesive and seeds.

The first filter and/or the second filter may be made of a biodegradable material, such as cellulose fibres, bioplastics or other degradable polymers, or a combination hereof. Alternatively, the first filter and/or second filter may be made of polypropylene (PP) or polyurethane (PUR) or the like.

The substance chamber and/or the evaporation chamber, e.g., the first cylindrical shell and/or the second cylindrical shell may be made of a biodegradable material, such as bioplastics, such as starch made of rice or corn. The biodegradable material may be edible and/or non-toxic. Alternatively, the substance chamber and/or the evaporation chamber may be made of polypropylene (PP) or the like.

The rod may be made of a biodegradable material, such as wood, compressed sawdust cement, pressed paper, cornmeal or wood cement.

Each of the first cylindrical shell, the second cylindrical shell, the first filter and the second filter may have an outer diameter which are substantially identical, e.g., after wrapping the wrapping sheet around the first cylindrical shell, the second cylindrical shell, the first filter and the second filter.

The first cylindrical shell may be symmetrical about a line halfway between the first end and the second end. Providing a symmetrical first cylindrical shell of the substance chamber simplifies the manufacturing of the inhalator comprising the substance chamber, as it may be arranged in either direction when arranging it relative to the evaporation chamber and/or other of the inhalator parts.

The first cylindrical shell may at the first end comprise a first connection portion. The first connection portion may be adapted to connect the substance chamber with a second part of the inhalator, such as the evaporation chamber. The second cylindrical shell of the evaporation chamber may at the second end comprise a second connection portion. The first connection portion at the first end of the first cylindrical shell may engage, e.g. in an interlocking configuration, with the second connection portion at the second end of the second cylindrical shell. The substance chamber and the second inhalator part, e.g. the evaporation chamber, may be mechanically interlocked. The connection portions provide for a connection between substance chamber and the evaporation chamber, such that the substance within the substance chamber may be transferred to the evaporation chamber with reduced risk of leakage of the substance.

The first cylindrical shell may at the second end comprise a second connection portion. The second connection portion of the first cylindrical shell may be adapted to connect the substance chamber with the second part, e.g. the evaporation chamber and/or a third part of the inhalator, e.g. the first filter. The first connection portion and the second connection portion of the first cylindrical shell may be identical. Thereby, the substance chamber may be arranged in either direction when arranging it relative to the evaporation chamber and/or other parts of the inhalator parts.

The second connection portion of the first cylindrical shell may abut to a first connection portion of the first filter. The second connection portion of the first cylindrical shell may be received in a first connection portion of the first filter or vice versa.

The wall thickness of the first cylindrical shell may differ within the first connection portion and/or within the second connection portion of the first cylindrical shell, relative to the wall thickness of the remainder of the first cylindrical shell, e.g. relative to the wall thickness of the first cylindrical shell at a point halfway between the first end and the second end of the first cylindrical shell. The differing wall thickness within the first connection portion and/or within the second connection portion of the first cylindrical shell may provide for connection of the substance chamber with the second part and/or the third part of the inhalator.

The outer diameter of the first cylindrical shell may decrease towards the first end within the first connection portion. The outer diameter of the first cylindrical shell may decrease towards the second end within the second connection portion. The outer diameter of the first cylindrical shell may decrease gradually within the first connection portion and/or within the second connection portion. The first connection portion and/or the second connection portion may be tapered. The outer diameter of the first cylindrical shell may decrease stepwise and/or in steps within the first connection portion and/or within the second connection portion. For example, the outer diameter of the first cylindrical shell may, within the first connection portion and/or within the second connection portion, decrease, e.g. in a single step, to be the same as the inner diameter of the second cylindrical shell.

The first connection portion of the first cylindrical shell may have a first connection portion length along the longitudinal axis. The second connection portion of the first cylindrical shell may have a second connection portion length along the longitudinal axis. The first connection portion length and/or the second connection portion length may be the same or may be longer than the wall thickness of the first cylindrical shell.

The first connection portion and/or the second connection portion of the first cylindrical shell may be shaped such that an inner surface of the first cylindrical shell and an outer surface of the first cylindrical shell tapers towards a point between the inner and outer surface to form a tipped end.

The first connection portion and/or the second connection portion of the first cylindrical shell may comprise a female connector configured to connect with a male connector of the second and/or third part of the inhalator. However, preferably, the first connection portion and/or the second connection portion of the first cylindrical shell may comprise a male connector configured to connect with a female connector of the second and/or third part of the inhalator.

The substance chamber may comprise a third sealing body movably arranged inside the first cylindrical shell between the first sealing body and the second sealing body. The first sealing body and the third sealing body may form a primary substance compartment of the substance compartment containing the primary substance. The second sealing body and the third sealing body may form a secondary substance compartment of the substance compartment containing a secondary substance. The third sealing body prevents mixing of the primary substance and the secondary substance in an inoperable state, which may be an advantage and/or requirement of the inhalator.

The substance chamber may comprise a spacer element, such as a first spacer element and/or a second spacer element. The substance chamber may comprise a first spacer element arranged inside the first cylindrical shell, e.g. between the first sealing body and the second sealing body and/or between the first sealing body and the third sealing body. The substance chamber may comprise a first spacer element arranged inside the first cylindrical shell, e.g. between the first sealing body and the second sealing body. The substance chamber may comprise a second spacer element arranged inside the first cylindrical shell, e.g. between the second sealing body and the third sealing body. The spacer elements may provide a space between the sealing bodies, such that the sealing bodies maintain their relative spacing during advancement of the sealing bodies and the substance of the substance chamber into the evaporation chamber to transition the inhalator from an inoperable state to an operable state.

The first spacer element and/or the second spacer element may have a non-circular cross section in a plane normal to the longitudinal axis. The first spacer element and/or the second spacer element may have a cross section in the plane normal to the longitudinal axis being cross shaped, being star shaped, or being a shape with two or more legs extending from a centre point. The first spacer element and/or the second spacer element may be formed such that the substance can be arranged in the cavity/cavities formed between the spacer element, the sealing bodies and the first cylindrical shell.

The first spacer element and/or the second spacer element may have a first width perpendicular to the longitudinal axis. The first width may be larger than half of the inner diameter of the first cylindrical shell. The first spacer element and/or the second spacer element may have a second width perpendicular to the longitudinal axis and perpendicular to the first width. The second width may be larger than half of the inner diameter of the first cylindrical shell. The width(s) of the first spacer element and/or the second spacer element may be such that it at least extends to the longitudinal axis (e.g. the centre of the first cylindrical shell). The size of the first spacer element and/or the second spacer element may be such that spacer element spaces the sealing bodies apart.

Further, the spacer element(s) may during manufacturing of the substance chamber assist by maintaining a specified space between the sealing bodies when the sealing bodies, the spacer element(s) and substance are pushed into place. In particular, the manufacturing of the substance chamber may comprise providing the first sealing body inside the first cylindrical shell, subsequently providing a spacer element inside the first cylindrical shell, e.g. pushing the first sealing body further inside the first cylindrical shell, filling the substance around and/or on the spacer element, and subsequently providing the second sealing body and/or the third sealing body inside the first cylindrical shell, thereby pushing the spacer element, the substance and the first sealing body further into the first cylindrical shell. The spacer element(s) prevents build-up of pressure between the sealing bodies when advancing the second sealing body and/or the third sealing body into the first cylindrical shell.

The spacer element(s) may be made of a biodegradable material, such as wood, compressed sawdust cement, pressed paper, cornmeal or wood cement.

The first sealing body may be arranged closer to the first end of the first cylindrical shell than the second sealing body and/or the third sealing body. The second sealing body may be arranged closer to the second end of the first cylindrical shell than the first sealing body. The third sealing body may be arranged closer to the second end of the first cylindrical shell than the first sealing body.

The sealing bodies, such as the first, the second and/or the third sealing body, are preferably formed as spheres. The sealing bodies may each comprise a diameter which is at least equal to the inner diameter of the first cylindrical shell. The sealing bodies may be slightly oversized spheres, i.e. slightly larger than the inner diameter of the first cylindrical shell. The sealing bodies may be silicone spheres or balls e.g. formed of rubber, plastic or silicone. The sealing bodies may thereby create a diffusion tight seal between the compartments - and thereby may prevent the substance(s) from leaking out of the substance chamber and preventing air from entering the substance chamber.

The method may comprise providing a plurality of inhalator parts, where providing the plurality of inhalator parts may comprise providing a second substance chamber according to the substance chamber as disclosed in the present disclosure, e.g. identical to the first substance chamber. The method may comprise arranging the first substance chamber, the second substance chamber and the first filter or the second filter coaxially along the longitudinal axis and such that each of the first and second substance chambers are arranged at each end of the first filter or the second filter. Providing the plurality of inhalator parts may comprise providing a second evaporation chamber, e.g. identical to the first evaporation chamber. The method may comprise arranging the first evaporation chamber, the second evaporation chamber, the first substance chamber, the second substance chamber and the first filter or the second filter coaxially along the longitudinal axis and such that each of the first evaporation chamber and the second evaporation chambers are arranged on each side of the first and second substance chambers. The method may comprise providing a wrapping sheet with a first surface, e.g. comprising adhesive on the first surface. The method may comprise wrapping the wrapping sheet circumferentially around the longitudinal axis and such that the first surface is facing and encircling the first filter or the second filter, the first substance chamber, the second substance chamber, the first evaporation chamber, and the second evaporation chamber. The method may comprise, e.g. after wrapping the wrapping sheet circumferentially around the longitudinal axis, cutting the first filter or the second filter and the wrapping sheet along a line perpendicular to the longitudinal axis and halfway between the first end and the second end of the first filter or the second filter. Thereby two inhalators may be manufactured in one manufacturing process, wherein the cut filter forms the first filter or the second filter, as described above. It should be understood that all elements of the inhalator, as described above, may be included in an inhalator manufactured in such dual manufacturing process.

The longitudinal axis may run in the longitudinal direction. The longitudinal axis of each of the plurality of inhalator parts may be the same and/or may coincide.

The inhalator may have the same or similar size as conventional cigarettes. All the inhalator parts may be of a non-toxic material.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Figs. 1a-1b are schematic diagrams illustrating an exemplary inhalator,
Figs. 2a-2f are schematic diagrams illustrating exemplary inhalator parts,
Figs. 3a-3d are schematic diagrams illustrating exemplary inhalator parts,
Figs. 4a-4e are schematic diagrams illustrating exemplary inhalator parts,
Figs. 5a-5b are schematic diagrams illustrating exemplary inhalator parts, and
Fig. 6 is schematic diagram illustrating exemplary method of manufacturing exemplary inhalators.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

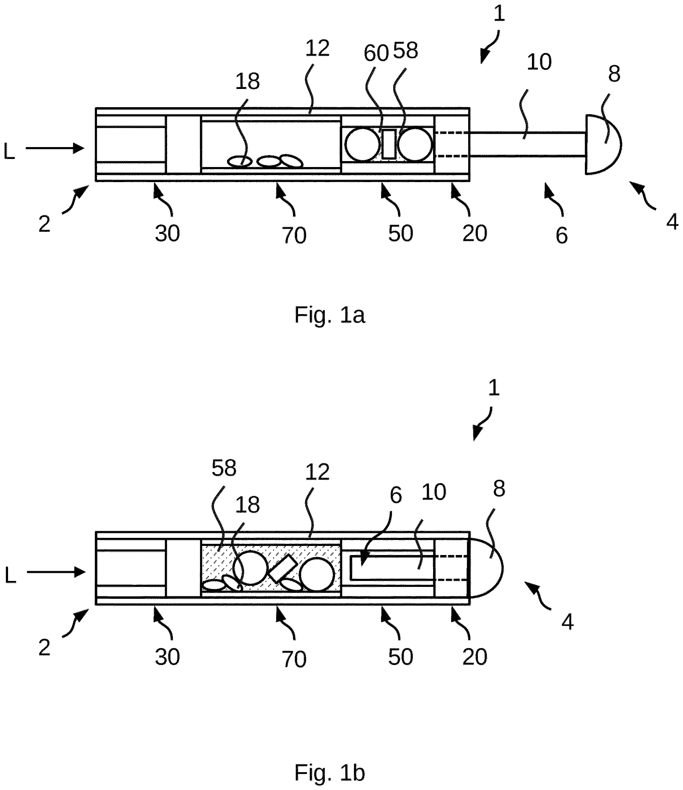

Figs. 1a-1b are schematic diagrams illustrating an exemplary inhalator 1 of the present disclosure.

Fig. 1a illustrates an assembled inhalator 1 which is in an inoperable state (e.g. a storage state) and

Fig. 1b illustrates an assembled inhalator 1 which is in an operative state.

The inhalator 1 extends along a longitudinal axis L from a first end 2 to a second end 4. The inhalator 1 comprises a plurality of inhalator parts comprising at least a substance chamber 50 and an evaporation chamber 70 arranged coaxially along the longitudinal axis L. The inhalator 1 may further comprise a rod 6, a first filter 20 and a second filter 30. A wrapping sheet 12 with a first surface 14, and which may comprise an adhesive 16 on the first surface 14, may encircle the plurality of inhalator parts 6, 20, 50, 70, 30 such that the first surface 14 faces the inhalator parts 6, 20, 50, 70, 30 and such that the inhalator parts 6, 20, 50, 70, 30 are maintained in their respective positions. The outer diameter of the inhalator parts 20, 50, 70, 30 are substantially the same.

The substance chamber 50 comprises a substance 58 which is enclosed in a substance compartment 60 in the inoperable state (Fig. 1a) and released into the evaporation chamber 70 in the operative state (Fig. 1b) by applying a force to the rod 6 towards the first end 2. The rod 6 allows air to pass from the second end 4 into the inhalator 1 in the operative state, e.g., through openings in the rod head 8, or by the rod head 8 having a diameter which is smaller than outer diameter of the first filter 20 to allow air to pass through the first filter around the rod head 8. The length of the rod 6 is larger than the length of the first filter 20 in the longitudinal direction and/or the length of the substance chamber 50 in the longitudinal direction.

The evaporation chamber 70 may comprise pellets 18 to increase the surface area within the evaporation chamber 70 and thereby improve evaporation of the substance 58. A user inhales the vaporized substance 58 with the user's mouth at the first end 2 of the inhalator. The first end 2 may comprise a mouthpiece (not shown).

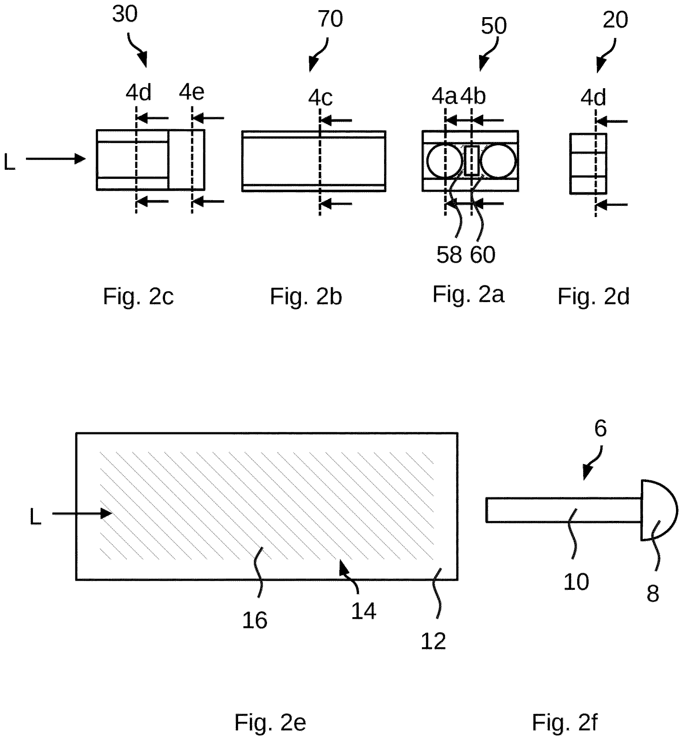

Figs. 2a-2f are schematic diagrams illustrating exemplary inhalator parts. Fig. 2a illustrates a substance chamber 50, such as the substance chamber 50 of Figs. 1a-1b. Fig. 2b illustrates an evaporation chamber 70, such as the evaporation chamber 70 of Figs. 1a-1b. Fig. 2c illustrates a second filter 30, such as the second filter 30 of Figs. 1a-1b. Fig. 2e illustrates a first filter 20, such as the first filter 20 of Figs. 1a-1b. Fig. 2e illustrates a wrapping sheet 12, such as the wrapping sheet 12 of Figs. 1a-1b. Fig. 2f illustrates a rod 6, such as the rod 6 of Figs. 1a-1b.

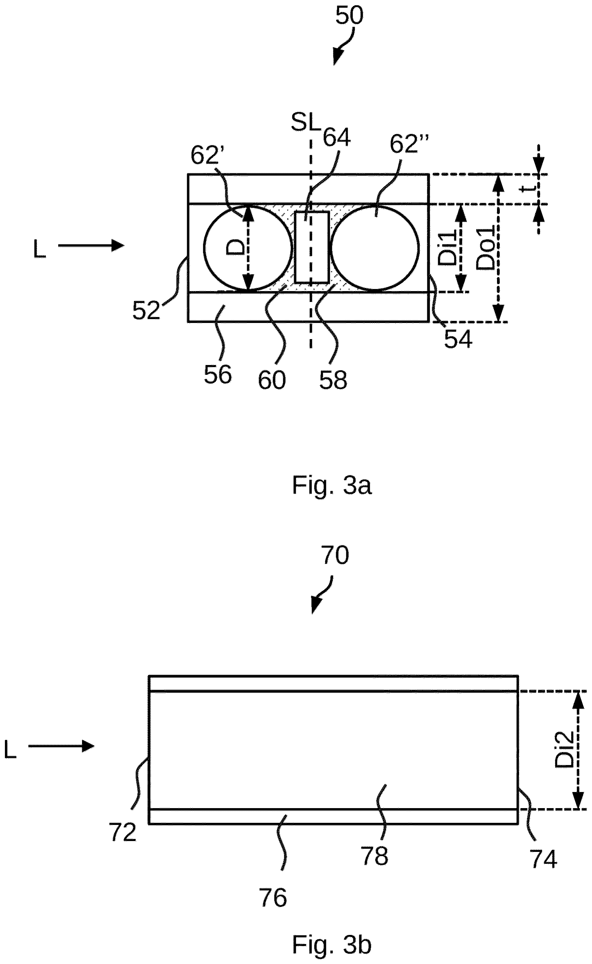

Fig. 3a illustrates a substance chamber 50, such as the substance chamber 50 of Figs. 1a-1b. The substance chamber 50 comprises a first cylindrical shell 56 which extends along a longitudinal axis L from a first end 52 to a second end 54. The first cylindrical shell 56 has an inner diameter Di1 and an outer diameter Do1 and a wall thickness t, which is half of the difference between the outer diameter Do1 and the inner diameter Di1. The substance chamber 50 may be symmetrical about a line SL halfway between the first end 52 and the second end 54.

The substance chamber 50 comprises a first sealing body 62' and a second sealing body 62" which are movably arranged inside the first cylindrical shell 56. The first sealing body 62' and a second sealing body 62" each has a diameter D. The diameter D is at least equal to the inner diameter of the first cylindrical shell 56. The first cylindrical shell 56, the first sealing body 62' and the second sealing body 62" form a substance compartment 60 containing a substance 58, such as a liquid substance, e.g. comprising nicotine and/or aromatic substances. A spacer element 64, such as a first spacer element may be arranged in the substance compartment 60 between the first sealing body 62' and the second sealing body 62". The spacer element 64 is provided to mechanically space the first sealing body 62' and the second sealing body 62" apart, such that, when a user wants to transition the inhalator 1 from the inoperable state to the operable state by pushing the sealing bodies 62', 62" into the evaporation chamber 70, a space corresponding to the spacer element 64 is maintained during the transition, and pressure buildup in the substance compartment 60 during the transition may be reduced. Further, the spacer element 64 assists in manufacturing of the substance chamber 50 by maintaining a space between the sealing bodies 62', 62" when the sealing bodies 62', 62", the spacer element 64, and the substance 58 are pushed into place, corresponding to the inoperable state.

Fig. 3b illustrates an evaporation chamber 70, such as the evaporation chamber 70 of Figs. 1a-1b. The evaporation chamber 70 comprises a second cylindrical shell 76 which extends along a longitudinal axis L from a first end 72 to a second end 74.

The length of the first cylindrical shell 56 is smaller than the length of the second cylindrical shell 76. The inner diameter Di1 of the first cylindrical shell 56 is smaller than the inner diameter Di2 of the second cylindrical shell 76. Thereby, when the sealing bodies 62', 62" are pushed into the second cylindrical shell (e.g. in the operative state), the substance 58 is released from the substance compartment 60.

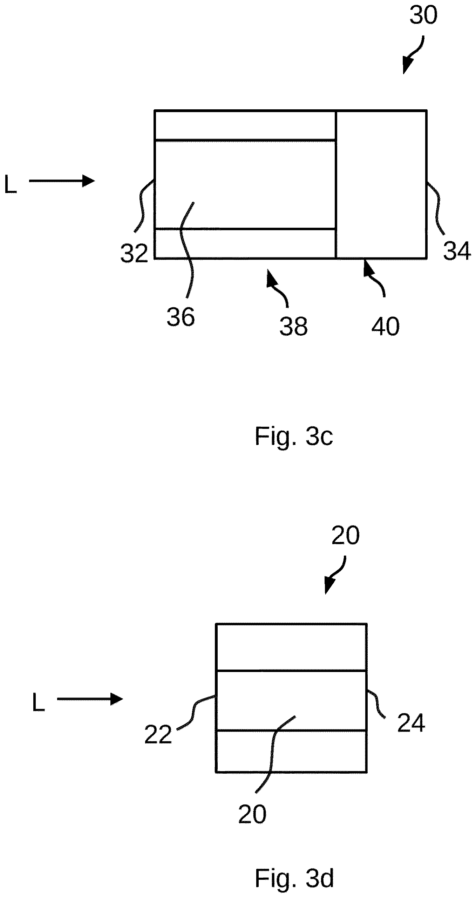

Fig. 3c illustrates a second filter 30, such as the second filter 30 of Figs. 1a-1b. The second filter 30 extends along a longitudinal direction L from a first end 32 to a second end 34 and comprises a first portion 38 at the first end 32 and a second portion 40 at the second end 34. The first portion 38 comprises a through hole 36 extending along the longitudinal axis L to allow air to pass through and to increase the amount of evaporated substance for intake for the user. The second portion 40 is devoid of a through hole. The second portion 40 may have a substantially continuous cross section. The second portion 40 may have a porous, multi-cellular cross-section, e.g., may comprise an open-celled material. The second portion 40 prevents the substance 58 in liquid form and/or the pellets 18 from escaping the evaporation chamber 70 while allowing substance 58 in evaporated form to pass.

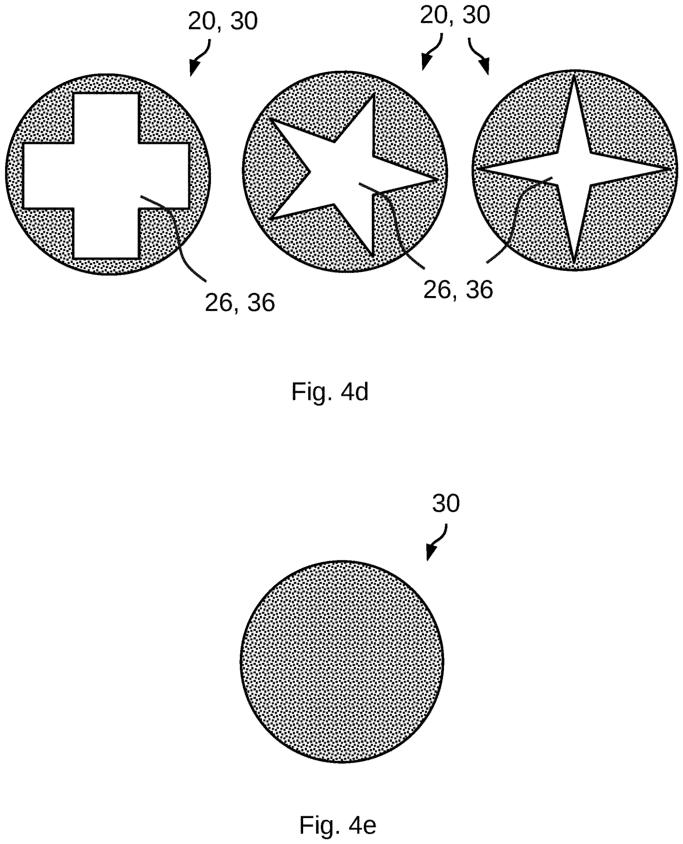

Fig. 3d illustrates a first filter 20, such as the first filter 20 of Figs. 1a-1b. The first filter 20 extends along a longitudinal direction L from a first end 22 to a second end 24 and comprises a through hole 26. The through hole 26 has a cross-section (see Fig. 4d) that allows the rod 6 to engage and extend through the first filter 20.

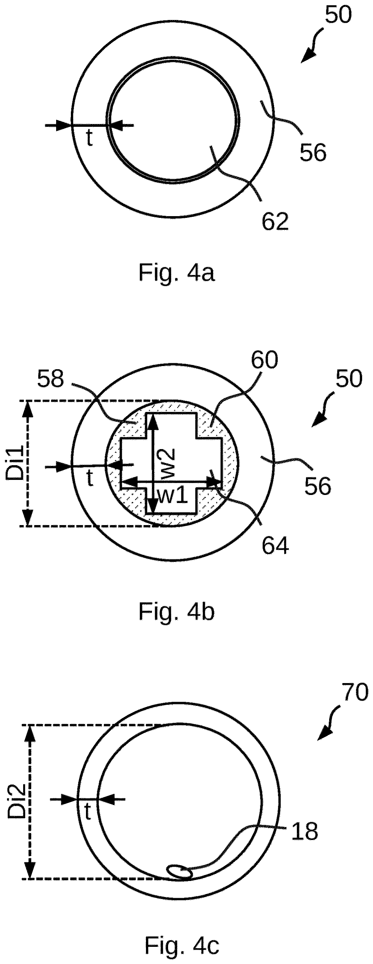

Figs. 4a-4e illustrate cross-sections of inhalator parts 50, 70, 20, 30, such as the inhalator parts of Figs. 3a-3d. The cross-sections are illustrated from locations corresponding to the lines 4a-4e in Figs. 2a-2d.

Figs. 4a and 4b show cross-sections of the substance chamber 50 at two different locations. Fig. 4a shows the cross-section through a location where the sealing bodies 62 are largest and the figure illustrates that the sealing bodies are arranged in a tight fit within the first cylindrical shell 56. For illustrative purposes only a minor gap is provided between the inner surface of the cylindrical shell 56 and the outer surface of the sealing body 62. Fig. 4b shows the cross-section through a location comprising a spacer element 64. The spacer element 64 has a shape (in the illustrated example, the spacer element 64 has a cross shaped cross-sectional shape) which allows storage of the substance 58 in the substance compartment 60 between the spacer element 64 and the first cylindrical shell 56. The spacer element 64 has a first width w1 perpendicular to the longitudinal axis which is larger than half of the inner diameter Di1 of the first cylindrical shell 56. The spacer element 64 has a second width w2 perpendicular to the longitudinal axis L and perpendicular to the first width w1 which is also larger than half of the inner diameter of the first cylindrical shell 56. The shape of the spacer element 64 may be non-circular, such as cross-shaped (as illustrated), star-shaped or rectangular. The function of the spacer element 64 may be realized through other shapes than the shape illustrated in Fig. 4b or the aforementioned shapes.

Fig. 4c shows the cross-section of the evaporation chamber 70. The evaporation chamber 70 may comprise one or more pellets 18.

Fig. 4d shows examples of the cross-section of the first filter 20 and/or of the second filter 30 at the first portion 38. The first filter 20 and/or the second filter 30 at the first portion 38 may have a similar cross-section. However, in some cases the first filter 20 and/or the second filter 30 at the first portion 38 may attain different cross-sectional shapes and/or may have different dimensions. The filters 20, 30 comprise a through hole 26, 36. The through hole 26 of the first filter 20 allows the rod to engage with and extend through the first filter 20. The rod 6, e.g., the rod body 10, may have a cross-section corresponding to the shape of through hole 26 or at least the rod 6, e.g. the rod body 10 may have a size and/or shape capable of being engaged in the through hole 26. The through hole 36 of the first portion 38 of the second filter 30 creates a passage for the evaporated substance 58 to pass. The shape of the through hole 36 may accommodate an increased amount of evaporated substance 58, e.g., by having a large through hole 36 to increase the volume through which the evaporated substance 58 passes or a long through hole 36 to create a specific flow pattern. The cross-section of the first filter 20 and/or the second filter 30 at the first portion 38 may be the same or may be different.

Fig. 4e shows an exemplary cross-section of the second filter 30 at the second portion 40. The second portion 40 of the second filter 30 comprises a substantially continuous cross section. The second portion comprises a porous and vapor permeable material. The second portion 40 of the second filter 30 prevents the substance 58 and/or the pellets 18 from escaping the evaporation chamber 70 while allowing evaporated substance 58 to pass through.

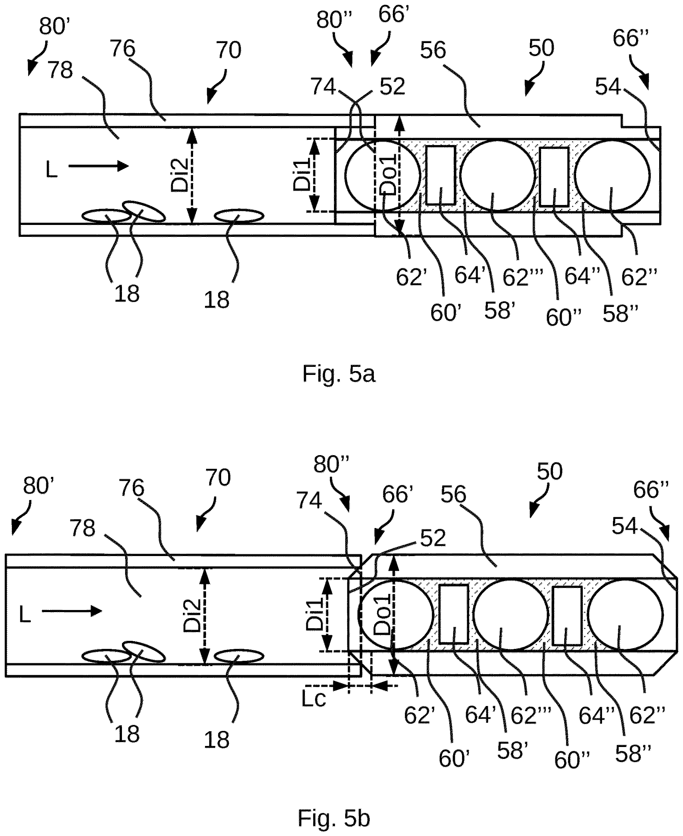

Fig. 5a and 5b schematically illustrate a plurality of inhalation parts, such as the substance chamber 50 and the evaporation chamber 70. The substance chamber 50 comprises a first connection portion 66' at the first end 52 and a second connection portion 66" at the second end 54. The first connection portion 66' and the second connection portion 66" may be the same or different. The substance chamber 50 and the evaporation chamber 70 are connected by the first connection portion 66' at the first end 52 of the first cylindrical shell 56 engaging with a second connection portion 80" at the second end 74 of the second cylindrical shell 76.

The outer diameter Do1 of the first cylindrical shell 56 decreases towards the first end 52 within the first connection portion 66'. Fig. 5a illustrates a stepwise decrease of the outer diameter Do, whereas Fig. 5b illustrates a gradual or tapered decrease of the outer diameter Do. The step of the first connection portion 66' of Fig. 5a may have a size which corresponds to the wall thickness of the second cylindrical shell 76. The first connection portion 66' has a first connection portion length Lc along the longitudinal axis which may be the same or may be longer than the wall thickness t of the first cylindrical shell 56.

The first connection portion 66' of the first cylindrical shell 56 may comprises a male connector, e.g. a male connector configured to connect to a female connector of the second cylindrical shell 76 or vice versa.

Figs. 5a and 5b further illustrate that the substance chamber 50 may comprise a third sealing body 62‴ and a second spacer element 64", which allows for the substance chamber to store both a primary substance 58' in a primary substance compartment 60' and a secondary substance 58" in a secondary substance compartment 60". The secondary substance 58" may be the same or different from the primary substance 58' stored in the primary substance compartment 60'.

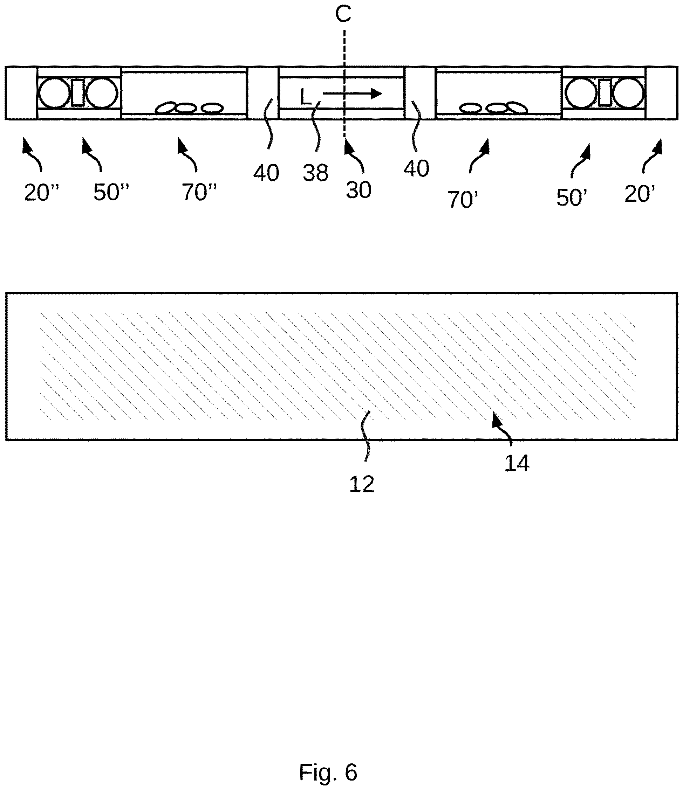

The inhalator may be manufactured in a high-speed and high-volume method as illustrated in Fig. 6. The method may comprise providing a second filter 30 comprising a first portion 38 and two second portions 40, one in each end. The method may comprise providing a first and second substance chamber 70', 70", a first and second evaporation chamber 50', 50" and a primary first filter 20' and a secondary first filter 20".

The method may comprise arranging the first evaporation chamber 70', the second evaporation chamber 70" and the second filter 30 coaxially along the longitudinal axis L and such that each of the first and second evaporation chambers 70', 70" are arranged at each end of the second filter 30.

The method may comprise arranging the first substance chamber 50', the second substance chamber 50", the first evaporation chamber 70', the second evaporation chamber 70" and the second filter 30 coaxially along the longitudinal axis L and such that each of the first substance chamber 50' and the second substance chambers 50" are arranged on each side of the first and second evaporation chambers 70', 70".

The method may comprise arranging the primary first filter 20', the secondary first filter 20", the second substance chamber 50", the first evaporation chamber 70', the second evaporation chamber 70" and the second filter 30 coaxially along the longitudinal axis L and such that each of the primary first filter 20' and the secondary first filter 20" arranged on each side of the first and second substance chambers 50', 50".

The method may comprise providing a wrapping sheet 12 and wrapping the wrapping sheet 12 circumferentially around the longitudinal axis L and such that the first surface 14 is facing and encircling the second filter 30, the first substance chamber 50', the second substance chamber 50', the first evaporation chamber 70', the second evaporation chamber 70", the primary first filter 20' and the secondary first filter 20".

The method may comprise cutting the second filter 30 and the wrapping sheet 12 along a line C perpendicular to the longitudinal axis L at a line halfway between the first end and the second end of the second filter 30, thereby providing two inhalators on each side of the cutting line C.

The disclosure has been described with reference to a preferred embodiment. However, the scope of the invention is not limited to the illustrated embodiment, and alterations and modifications can be carried out without deviating from the scope of the invention.

Throughout the description, the use of the terms "first", "second", "third", "fourth", "primary", "secondary", "tertiary" etc. does not imply any particular order or importance but are included to identify individual elements. Furthermore, the labelling of a first element does not imply the presence of a second element and vice versa.

### ITEMS

1. A substance chamber for an inhalator comprising the substance chamber and an evaporation chamber, wherein the substance chamber comprises:
   - a first cylindrical shell extending in a longitudinal axis from a first end to a second end, and having an inner diameter, an outer diameter, and a wall thickness being half of the difference between the outer diameter and the inner diameter,
   - a first sealing body and a second sealing body movably arranged inside the first cylindrical shell,
   wherein the first cylindrical shell, the first sealing body and the second sealing body form a substance compartment containing at least a primary substance, preferably a liquid substance.
2. Substance chamber according to item 1, wherein the first cylindrical shell is symmetrical about a line halfway between the first end and the second end.
3. Substance chamber according to any of the preceding items, wherein the first cylindrical shell at the first end comprises a first connection portion adapted to connect the substance chamber with a second part of the inhalator.
4. Substance chamber according to item 3, wherein the first cylindrical shell at the second end comprises a second connection portion adapted to connect the substance chamber with the second part and/or a third part of the inhalator.
5. Substance chamber according to item 4, wherein the first connection portion and the second connection portion of the first cylindrical shell are identical.
6. Substance chamber according to any of items 3-5, wherein the outer diameter of the first cylindrical shell decreases towards the first end within the first connection portion.
7. Substance chamber according to item 3-6, wherein the first connection portion has a first connection portion length along the longitudinal axis, and wherein the first connection portion length is the same or longer than the wall thickness of the first cylindrical shell.
8. Substance chamber according to any of items 3-5, wherein the outer diameter of the first cylindrical shell decreases towards the second end within the second connection portion.
9. Substance chamber according to any of items 3-7, wherein the first connection portion of the first cylindrical shell is shaped such that an inner surface of the first cylindrical shell and an outer surface of the first cylindrical shell tapers towards a point between the inner and outer surface to form a tipped end.
10. Substance chamber according to any of items 3-9, wherein the first connection portion and/or the second connection portion of the first cylindrical shell comprises a male connector configured to connect to a female connector of the second and/or third part of the inhalator.
11. Substance chamber according to any of items 3-10, wherein the wall thickness of the first cylindrical shell may differ within the first connection portion and/or within the second connection portion of the first cylindrical shell relative to the wall thickness of the remainder of the first cylindrical shell.
12. Substance chamber according to any of the preceding items comprising a first spacer element arranged inside the first cylindrical shell between the first sealing body and the second sealing body.
13. Substance chamber according to item 12, wherein the first spacer element has a non-circular cross section in a plane normal to the longitudinal axis.
14. Substance chamber according to any of items 12-13, wherein the first spacer element has a first width perpendicular to the longitudinal axis, wherein the first width is larger than half of the inner diameter of the first cylindrical shell.
15. Substance chamber according to item 14, wherein the first spacer element has a second width perpendicular to the longitudinal axis and perpendicular to the first width, wherein the second width is larger than half of the inner diameter of the first cylindrical shell.
16. Substance chamber according to any of the preceding items comprising a third sealing body movably arranged inside the first cylindrical shell between the first sealing body and the second sealing body, the first sealing body and the third sealing body forms a primary substance compartment of the substance compartment containing the primary substance, and the second sealing body and the third sealing body forms a secondary substance compartment of the substance compartment containing a secondary substance.
17. Substance chamber according to item 16 comprising a second spacer element arranged inside the first cylindrical shell between the second sealing body and the third sealing body.
18. Substance chamber according to any of the preceding items wherein the first sealing body is arranged closer to the first end of the cylindrical shell than the second sealing body and/or the second sealing body is arranged closer to the second end of the cylindrical shell than the first sealing body.
19. Substance chamber according to any of the preceding items wherein the first sealing body and/or the second sealing body are spherical.
20. Substance chamber according to any of the preceding items wherein the first sealing body and/or the second sealing body each comprises a diameter which is at least equal to the inner diameter of the first cylindrical shell.
21. An inhalator comprising a plurality of inhalator parts and extending from a first end to a second end along a longitudinal axis, the plurality of inhalator parts comprising:
   - the substance chamber according to any of the preceding items, wherein the longitudinal axis of the first cylindrical shell and the longitudinal axis of the inhalator are coinciding,
   - an evaporation chamber comprising a second cylindrical shell extending along the longitudinal axis from a first end to a second end, wherein an inner diameter of the second cylindrical shell is larger than the inner diameter of the first cylindrical shell of the substance chamber,
   wherein the substance chamber and the evaporation chamber are arranged coaxially along the longitudinal axis, and wherein the first end of the first cylindrical shell is arranged towards the second end of the second cylindrical shell.
22. Inhalator according to item 21, wherein the first connection portion at the first end of the first cylindrical shell engages, e.g. in an interlocking configuration, with a second connection portion at the second end of the second cylindrical shell.
23. Inhalator according to any of the items 21-22 comprising:
   - a rod extending from a first end to a second end along the longitudinal axis,
   - a first filter extending from a first end to a second end along the longitudinal axis,
      wherein the rod and the first filter are arranged coaxially along the longitudinal axis and such that the first end of the rod is arranged towards the second end of the first filter and wherein the rod is configured to be received in the first filter,
      wherein the first filter and the first cylindrical shell are arranged coaxially along the longitudinal axis and such that the first end of the first filter is arranged towards the second end of the first cylindrical shell.
24. Inhalator according to any one of the items 21-23 comprising a second filter extending from a first end to a second end along the longitudinal axis, wherein the second filter and the second cylindrical shell are arranged coaxially along the longitudinal axis, and wherein the second end of the second filter is arranged towards the first end of the second cylindrical shell.
25. Inhalator according to item 24, wherein the second filter comprises a first portion at the first end of the second filter and a second portion at the second end of the second filter, and wherein the first portion comprises a through hole extending along the longitudinal axis and/or wherein the first portion and/or the second portion has a substantially continuous cross section.
26. Inhalator according to any one of the items 21-25 comprising a wrapping sheet, wherein the wrapping sheet is wrapped circumferentially around the longitudinal axis and encircling the substance chamber, the evaporation chamber, the first filter and the second filter.
27. Inhalator according to item 26, wherein the wrapping sheet comprises an adhesive on a first surface, and wherein the first surface is facing the plurality of inhalator parts.
28. Inhalator according to any one of the items 21-27, wherein the second connection portion of the first cylindrical shell abuts to a first connection portion of the first filter.
29. Inhalator according to any one of the items 21-28, wherein the length of the rod along the longitudinal axis is larger than the length of the first cylindrical shell along the longitudinal axis.
30. Inhalator according to any one of the items 21-26, wherein each of the first cylindrical shell, the second cylindrical shell, the first filter and the second filter has an outer diameter which are substantially identical.
31. Inhalator according to any one of the items 21-30, wherein the evaporation chamber comprises one or more pellets.
32. Inhalator according to any one of the items 21-30, wherein one or more of the substance chamber, evaporation chamber, rod, first filter, second filter or wrapping sheet comprises one or more pellets, such as seeds.
33. A method for manufacturing an inhalator comprising a plurality of inhalator parts and extending from a first end to a second end along a longitudinal axis, the method comprising the steps of:
   - providing the plurality of inhalator parts comprising:
      - providing a first substance chamber according to any one of the items 1-20, wherein the longitudinal axis of the first cylindrical shell and the longitudinal axis of the inhalator are coinciding,
      - providing a first evaporation chamber comprising a second cylindrical shell extending along the longitudinal axis from a first end to a second end, wherein an inner diameter of the second cylindrical shell is larger than the inner diameter of the first cylindrical shell of the substance chamber,
      - arranging the first substance chamber and the first evaporation chamber coaxially along the longitudinal axis, and such that the first end of the first cylindrical shell is arranged towards the second end of the second cylindrical shell,
      - providing a wrapping sheet with a first surface, e.g., comprising adhesive on the first surface, and
      - wrapping the wrapping sheet circumferentially around the longitudinal axis and such that the first surface is facing and encircling the first substance chamber and the first evaporation chamber.
34. The method according to item 33, wherein
   - providing the plurality of inhalator parts comprises providing a second filter extending from a first end to a second end along the longitudinal axis,
   and wherein the method comprises:
   - arranging the second filter and the first evaporation chamber coaxially along the longitudinal axis, and such that the second end of the second filter is arranged towards the first end of the second cylindrical shell.
35. The method according to any one of the items 33-34, wherein
   - providing the plurality of inhalator parts comprises providing a first filter extending from a first end to a second end along the longitudinal axis,
   and wherein the method comprises:
   - arranging the first filter and the first substance chamber coaxially along the longitudinal axis, and such that the second end of the first cylindrical shell is arranged towards the first end of first filter.
36. The method according to any one of the items 33-35, wherein
   - providing the plurality of inhalator parts comprises providing comprises providing a rod extending from a first end to a second end along the longitudinal axis and providing a first filter extending from a first end to a second end along the longitudinal axis and comprising a through hole, wherein the rod is received in the through hole of the first filter,
   and wherein the method comprises:
   - arranging the first filter and the first substance chamber coaxially along the longitudinal axis, and such that the second end of the first cylindrical shell is arranged towards the first end of first filter.
37. The method according to any one of the items 33-36, wherein providing the plurality of inhalator parts comprises:
   - providing a second substance chamber according to any one of the items 1-20,
   - providing a second evaporation chamber identical to the first evaporation chamber, the method further comprising:
   - arranging the first evaporation chamber, the second evaporation chamber and the second filter coaxially along the longitudinal axis and such that each of the first and second evaporation chambers are arranged at each end of the second filter,
   - arranging the first substance chamber, the second substance chamber, the first evaporation chamber, the second evaporation chamber and the second filter coaxially along the longitudinal axis and such that each of the first substance chamber and the second substance chambers are arranged on each side of the first and second evaporation chambers,

   wherein wrapping the wrapping sheet circumferentially around the longitudinal axis is performed such that the first surface is facing and encircling the second filter, the first substance chamber, the second substance chamber, the first evaporation chamber, and the second evaporation chamber,
   and after wrapping the wrapping sheet, cutting the second filter and the wrapping sheet along a line perpendicular to the longitudinal axis at a line halfway between the first end and the second end of the second filter.

### LIST OF REFERENCES

- 1: inhalator
- 2: first end
- 4: second end
- 6: rod
- 8: rod head
- 10: rod body
- 12: wrapping sheet
- 14: first surface
- 16: adhesive
- 18: pellets

- 20: first filter
- 20': primary first filter
- 20": secondary first filter
- 22: first end
- 24: second end
- 26: through hole

- 30: second filter
- 32: first end
- 34: second end
- 36: through hole
- 38: first portion
- 40: second portion

- 50: substance chamber
- 50': first substance chamber
- 50": second substance chamber
- 52: first end
- 54: second end
- 56: first cylindrical shell
- 58: substance
- 58': primary substance
- 58": secondary substance
- 60: substance compartment
- 60': primary substance compartment
- 60": secondary substance compartment
- 62: sealing body
- 62': first sealing body
- 62": second sealing body
- 62'": third sealing body
- 64: spacer element
- 64': first spacer element
- 64": second spacer element
- 66: connection portion
- 66': first connection portion
- 66": second connection portion

- 70: evaporation chamber
- 70': first evaporation chamber
- 70": second evaporation chamber
- 72: first end
- 74: second end
- 76: second cylindrical shell
- 78: evaporation compartment
- 80: connection portion
- 80': first connection portion
- 80": second connection portion

- L: longitudinal axis
- SL: symmetry line
- D: diameter
- Di1: inner diameter of first cylindrical shell
- Di2: inner diameter of second cylindrical shell
- Do1: outer diameter of first cylindrical shell
- t: wall thickness
- Lc: connection portion length
- w1: first width
- w2: second width
- C: cutting line

## Claims

1. Substance chamber for an inhalator comprising a substance chamber and an evaporation chamber, wherein the substance chamber comprises:
- a first cylindrical shell extending in a longitudinal axis from a first end to a second end, and having an inner diameter, an outer diameter, and a wall thickness being half of the difference between the outer diameter and the inner diameter,
- a first sealing body and a second sealing body movably arranged inside the first cylindrical shell,
wherein the first cylindrical shell, the first sealing body and the second sealing body form a substance compartment containing at least a primary substance, preferably a liquid substance.

2. Substance chamber according to claim 1, wherein the first cylindrical shell is symmetrical about a line halfway between the first end and the second end.

3. Substance chamber according to any of the preceding claims, wherein the first cylindrical shell at the first end comprises a first connection portion adapted to connect the substance chamber with a second part of the inhalator.

4. Substance chamber according to claim 3, wherein the first cylindrical shell at the second end comprises a second connection portion adapted to connect the substance chamber with the second part and/or a third part of the inhalator.

5. Substance chamber according to any of claims 3-5, wherein the outer diameter of the first cylindrical shell decreases towards the first end within the first connection portion.

6. Substance chamber according to any of the preceding claims comprising a first spacer element arranged inside the first cylindrical shell between the first sealing body and the second sealing body.

7. Substance chamber according to claim 6, wherein the first spacer element has a non-circular cross section in a plane normal to the longitudinal axis.

8. Substance chamber according to any of claims 6-7, wherein the first spacer element has a first width perpendicular to the longitudinal axis, wherein the first width is larger than half of the inner diameter of the first cylindrical shell and/or wherein the first spacer element has a second width perpendicular to the longitudinal axis and perpendicular to the first width, wherein the second width is larger than half of the inner diameter of the first cylindrical shell.

9. An inhalator comprising a plurality of inhalator parts and extending from a first end to a second end along a longitudinal axis, the plurality of inhalator parts comprising:
- the substance chamber according to any of the preceding claims, wherein the longitudinal axis of the first cylindrical shell and the longitudinal axis of the inhalator are coinciding,
- an evaporation chamber comprising a second cylindrical shell extending along the longitudinal axis from a first end to a second end, wherein an inner diameter of the second cylindrical shell is larger than the inner diameter of the first cylindrical shell of the substance chamber,
wherein the substance chamber and the evaporation chamber are arranged coaxially along the longitudinal axis, and wherein the first end of the first cylindrical shell is arranged towards the second end of the second cylindrical shell.

10. Inhalator according to claim 9, wherein the first connection portion at the first end of the first cylindrical shell engages, e.g. in an interlocking configuration, with a second connection portion at the second end of the second cylindrical shell.

11. Inhalator according to any of the claims 9-10 comprising:
- a rod extending from a first end to a second end along the longitudinal axis,
- a first filter extending from a first end to a second end along the longitudinal axis,
wherein the rod and the first filter are arranged coaxially along the longitudinal axis and such that the first end of the rod is arranged towards the second end of the first filter and wherein the rod is configured to be received in the first filter,
wherein the first filter and the first cylindrical shell are arranged coaxially along the longitudinal axis and such that the first end of the first filter is arranged towards the second end of the first cylindrical shell.

12. Inhalator according to any one of the claims 9-11 comprising a second filter extending from a first end to a second end along the longitudinal axis, wherein the second filter and the second cylindrical shell are arranged coaxially along the longitudinal axis, and wherein the second end of the second filter is arranged towards the first end of the second cylindrical shell.

13. Inhalator according to claim 12, wherein the second filter comprises a first portion at the first end of the second filter and a second portion at the second end of the second filter, and wherein the first portion comprises a through hole extending along the longitudinal axis and/or wherein the first portion and/or the second portion has a substantially continuous cross section.

14. Inhalator according to any one of the claims 9-13 comprising a wrapping sheet, wherein the wrapping sheet is wrapped circumferentially around the longitudinal axis and encircling the substance chamber, the evaporation chamber, the first filter and the second filter.

15. A method for manufacturing an inhalator comprising a plurality of inhalator parts and extending from a first end to a second end along a longitudinal axis, the method comprising the steps of:
- providing the plurality of inhalator parts comprising:
- providing a first substance chamber according to any one of the claims 1-19, wherein the longitudinal axis of the first cylindrical shell and the longitudinal axis of the inhalator are coinciding,
- providing a first evaporation chamber comprising a second cylindrical shell extending along the longitudinal axis from a first end to a second end, wherein an inner diameter of the second cylindrical shell is larger than the inner diameter of the first cylindrical shell of the substance chamber,
- arranging the first substance chamber and the first evaporation chamber coaxially along the longitudinal axis, and such that the first end of the first cylindrical shell is arranged towards the second end of the second cylindrical shell,
- providing a wrapping sheet comprising adhesive on a first surface, and
- wrapping the wrapping sheet circumferentially around the longitudinal axis and such that the first surface is facing and encircling the first substance chamber and the first evaporation chamber.
